# EUROPEAN PATENT APPLICATION

(11) **EP 2 581 847 A1**
(43) Date of publication of application: **17.04.2013**
(21) Application number: 11462020.6
(22) Date of filing: 13.10.2011
(51) Int. Cl.: G06F 19/00

(54) **Method for communication with individuals in a health care system**

(71) Applicant: General Electric Company, Schenectady, NY 12345 (US)
(72) Inventor: Balázs, Gábor, 6725 Szeged (HU); Engard, Ferenc, 2040 Budaörs (HU); Erdös, Akos, 2040 Budaörs (HU); Kiss, Bence, 3534 Miskolc (HU)
(74) Representative: Mak, Andras

(57) **Abstract**

A method is proposed for communication with a patient at a location distant from medical assistance, such as in a home environment for reminding the patient of activities relating to medication and/or carrying out measurements or other activities. The method comprises the steps of:
- providing a set of predetermined activities to be performed by the assisted individuals,
- providing a set of predetermined instructions relating to the predetermined activities to be communicated to the assisted individual,
- providing a set of predetermined rules for each of the activities in order to establish proper conditions for the activities to be carried out,
- continuous monitoring the behavior of the individuals for determining behavioral events in their daily routine,
- determining the proper timing conditions of the predetermined activities with relation to the behavioral events and
- communicating the instructions to the individuals according to the determined proper timing conditions. Also proposed is a system for performing the method.

## Description

### TECHNICAL FIELD

The invention generally relates to a method for communication with individuals in a health care or monitoring system, in particular in a remote health care or monitoring system, or more specifically to a method for determining the proper conditions for performing specific activities by the individuals.

### TECHNICAL BACKGROUND

Health monitoring of individuals, especially of elderly people in their homes is getting more and more important as hospitals are often overcrowded, too far or too expensive on a longer time basis. Many attempts have been made in the past in order to facilitate remote health care of elderly people, and to provide methods and systems for providing health monitoring data for care taking persons at different levels from medical specialists, through health care personnel to family members. On the basis of the collected information instructions may be given to the patients in order to carry our specific activities, relating to medication and/or carrying out measurements or other activities, such as rehabilitation exercises.

In a remote health care or monitoring system, the health condition of the patients in monitored by carrying out measurements continually or on a scheduled basis. US Pat. no. 5,917,414 discloses a body-worn monitoring system for obtaining and evaluating data from a person continuously in order to determine his/her daily routine, detect non-routine activity, and instruct the person to return to normal routine. US Pat. no. 5,967,975 describes a home health parameter monitoring system in which remote stations are connected to a central monitoring station through a communication link. Central station hardware/software verifies receipt of signals received from the remote stations within each of the subscriber's uniquely scheduled measurement time windows, and alerts alarm dispatchers to take prespecified action if a scheduled measurement is forgotten. In case of scheduled measurement it cannot be guaranteed that the desired circumstance is existent at the time when the scheduling is set for the measurement, however medical professionals monitoring the patients make their decisions based on these measurement results.

On the other hand, even though elderly people have usually a stable daily schedule, each of them has a different one, thus setting up the system with scheduled values can be vulnerable. Moreover the daily schedule can change for different reasons e.g.: because of diseases.

Due to the disadvantages of the prior art systems and methods there is a continuous need for providing a method and system which makes it possible to improve scheduling of activities of patients in a health care system on an individual basis.

### SUMMARY OF THE INVENTION

According to one aspect, a method is proposed for communication with an individual to be monitored at a location distant from medical assistance, such as in a home environment, in order to remind the individual of activities relating to medication and/or carrying out measurements or other activity, such as rehabilitation exercises. The method comprises the steps of:
- providing a set of predetermined activities to be performed by the assisted individuals,
- providing a set of predetermined instructions relating to the predetermined activities to be communicated to the assisted individual,
- providing a set of predetermined rules for each of the activities in order to establish proper conditions for the activities to be carried out,
- continuous monitoring the behavior of the individuals for determining behavioral events in their daily routine,
- determining the proper timing conditions of the predetermined activities with relation to the behavioral events and
- communicating the instructions to the individuals according to the determined proper timing conditions.

According to another aspect, a system is proposed for performing the method. A system for communication with an individual to be monitored at a location distant from medical assistance, such as in a home environment, in order to remind the individual of prescribed activities relating to medication and/or carrying out measurements or other activity, such as rehabilitation exercises, comprises:
- a plurality of subsystems at the location of the individuals to be monitored, the subsystems comprising a subsystem control unit and at least one behavioral event information collecting unit for collecting information from and relating to the monitored person, with at least a part of the information collecting units being capable of communicating with the subsystem control unit,
- a central data server station being capable of communicating with the subsystems, and
- monitoring side terminals being capable of communicating with the central station for providing information for health care professionals and/or care giving personnel and/or authorized family members,
- the subsystem control unit further comprising a communication storage for storing
   - a set of predetermined activities to be performed by the individuals,
   - a set of predetermined instructions relating to the predetermined activities to be communicated to the individual, and
   - a set of predetermined rules for each of the activities in order to establish proper conditions for the activities to be carried out,
- a program for controlling the communication with the individuals, on the basis of the stored data, characterized in that the system further comprises:
- at least one behavioral event analyzing unit for determining the proper timing conditions on the basis of the detected behavioral events.

In this invention the medical professionals can define the desired timing conditions and other circumstances for performing specific activities by the patients, because the scheduling is based on the patient's behavioral data in the home monitoring system and therefore it is possible to carry out all activities at the proper time or under the proper conditions.

### BRIEF DESCRIPTION OF THE DRAWINGS

The invention will now be described in detail with reference to the accompanying drawing, in which
- Fig. 1: is a schematic bock diagram of an embodiment of an extended system for performing remote monitoring,
- Fig. 2: is a schematic bock diagram of an embodiment of a monitoring subsystem,
- Fig. 3: is a schematic diagram of an embodiment of a behavioral monitoring system in the home environment and
- Fig. 4: is a schematic block diagram of detail of an embodiment of the subsystem.

### DESCRIPTION OF THE EMBODIMENTS

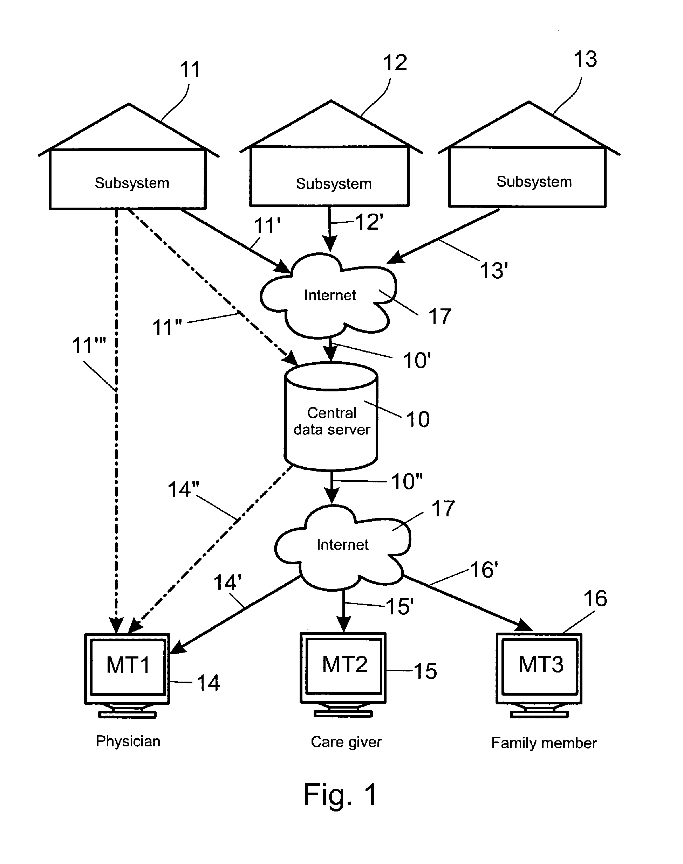

Referring first to Fig. 1, a schematic block diagram of a remote health monitoring system is shown. The system comprises a plurality of patient side subsystems 11, 12, 13 at the location of the individuals (or patients) to be monitored distant from medical assistance, such as in a home environment. In the simplest case a subsystem, such as subsystem 11 may be connected to a health care provider side subsystem, comprising monitoring terminals 14, 15 or 16. In the shown embodiment the subsystem 11 is connected to monitoring terminal 14 via a communication channel 11"'. The communication channel 11"' may be either a radio or a cable communication channel. In this case a monitoring person may have only access to one individual to be monitored at a time. Each change of the monitored person would require a reconnection to another communication channel. This problem can be solved by using a central communication and data server station 10 with a database, which is capable of communicating with the subsystems 11, 12, 13 via data communication channels, through a cable or an air interface. The monitoring terminals 14, 15, 16 are also capable of communicating with the central server station 10 in order to provide information for visual display to the monitoring persons, such as health care professionals and/or care giving personnel and/or authorized family members. Each group of the monitoring persons has a predetermined access right category to access monitoring information provided by the subsystems 11, 12, 13 and the central server unit 10. The health care professionals may for example be authorized to functionalities such as browsing patient data and setting up the monitoring parameters for the individual patient. The caregiver personnel may be authorized to browsing patient data and preparing different reports based on it. The family members may be authorized to have access to their respective relative in order to have information about his or her health condition. The monitoring terminals 14, 15, 16 may be connected to the central server 10 through either a radio communication channel or a cable communication channel, or a combination of a radio communication channel and a cable communication channel 14', 15', 16', such as the Internet 17. The use of the Internet as a communication channel makes it possible to set up the elements of the remote health monitoring system at any location of the world without any limitation. Therefore in a most flexible configuration, the elements of the system, e.g. the subsystems 11, 12, 13 are connected through communication links 11', 12', 13', the central server unit 10 through communication links 10' and 10", and the monitoring terminals 14, 15, 16 through communication links 14', 15', 16' to the Internet 27. In a general configuration of Fig. 1 the central server station 10 receives and stores all the data from the connected home hubs (subsystem control units) and provides access to information about the health condition of all individuals included in the system to the authorized monitoring persons.

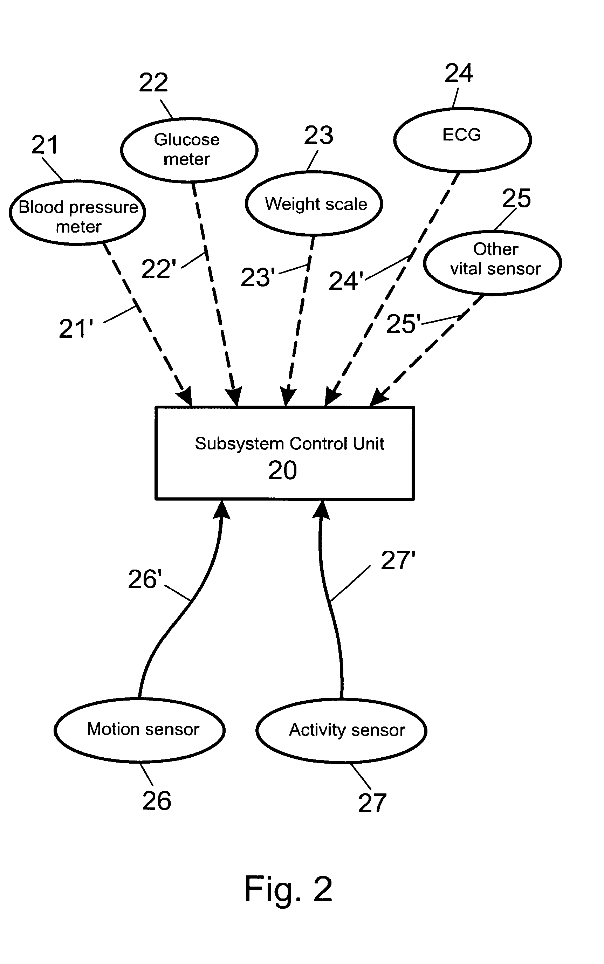

In this configuration of the health monitoring system for determining and applying different monitoring profiles for the different patients, the patient side subsystem 11 to 13 may also comprise a subsystem control unit 20 and measuring units for performing continuous measurement of behavioral data of the individuals to be monitored or distinct measurements of vital signs. In a patient side subsystem, as shown in Fig. 2, a subsystem control unit 20 (also called homehub) may be connected to measuring units 21 to 25 for measuring vital signs of the individuals to be monitored and to measuring units 26 and 27 for performing continuous measurement of behavioral data of the individuals to be monitored. The subsystem control unit further is able to receive, store and apply monitoring profiles provided by the central server unit.

The measuring units 21 to 25 for measuring vital signs have a communication link to the subsystem control unit 10 as indicated by the dotted lines 21' to 25'. These communication links may be accomplished by wired communication links or by wireless communication links. The measuring units for measuring vital signs of the individuals to be monitored may include for example but not exclusively, a blood pressure meter 21 for measuring the blood pressure, a glucometer 22 for measuring the blood glucose, weight scale 23 for determining the weight, an ECG monitor 24 for providing ECG data and other vital sign sensors 25 with the interaction of the monitored person. A part or all of these measuring units may have a wireless communication link to the subsystem control unit 20 as indicated by the dotted lines 21' to 25'. The wireless communication may be performed by using a radio communication according to the Bluetooth, Zigbee, Wifi or other standardized specifications. Some of the devices that are outside the wireless communication range or do not have a wireless communication capability, may be connected to the subsystem control unit 20 by a communication wire, such as a USB cable or the like. The vital sign measuring devices need not to be specialized devices, any measuring units available off-the shelf may be suitable for purposes of the invention, even those which do not have any connection possibility. In this case, the homehub used in the system must have a manual input capability, to enable the user to input the measuring result obtained from such a measuring unit, e.g. a bathroom weight scale. Using such a device the individual will read the result from the device and input (type) the value(s) using an input device (keyboard) of the homehub. Such a subsystem for assisting elderly people or patients in carrying out vital sign measurement on their own, are known in the prior art such as disclosed in US Patent no 7,684,999.

The subsystem control unit 20 is configured to receive and store the results of the distinct measurements of vital signs and of the continuous measurement of behavioral data. The measuring units 26 and 27 for performing continuous measurement of behavioral data of the individuals to be monitored may include for example some fix mounted motion sensors 26 for determining the motion and/or location of the individual in a selected area, and/or some body-worn sensors for sensing the activity such as speed and/or acceleration of the motion of a selected body part of the individual for determining the motion activity of the individual. The fix mounted motion sensors 26 may communicate with the subsystem control unit 20 using either a wireless or a wired communication link. In case of the body-worn sensors however it may be more advantageous if they are communicating with the subsystem control unit 20 using either through a wireless communication link so as not to restrict the wearer in his or her movement in any way. As a wired or wireless communication link, the same or similar communication link may be selected as the ones used for the measuring unit for measuring vital signs.

The fix mounted sensors may be for example motion detectors 26 or contact sensors mounted on walls or other pieces of furniture or equipments of the living area of the individuals to be monitored. These sensors are not in direct contact with the monitored person. The contact sensors can have different function in a monitored area. Typical installation points are the front door of the house/apartment, doors which might be useful to know if open or closed (e.g. bathroom door), doors of household equipment (e.g. door of the fridge) and critical places healthcare related monitoring (e.g. if the person keeps all the medication in a closed drawer or box, the door of this holder). Motion sensors, such as passive infrared sensors (PIR sensors) can measure infrared light radiating from objects in its field of view. Actual motion is detected when an infrared source with one temperature changes its position in front of an infrared source with another temperature, so in our case when a human passes the sensor's field of view in the monitored are. If there's a higher amount of motion then a pre-defined threshold, the sensor sends a signal to the subsystem control unit.

The body-worn sensor may be for example an activity sensor 27 such as a speed or acceleration sensor fixed to a part of the body, preferably to a hand or arm or to a leg or foot of the wearer. These sensors are in direct contact with the monitored person. The body-worn sensor or Actigraph as it is generally called, is a body-worn equipment, most often worn on the wrist, like a wristwatch. The unit continually records the movement of the equipment itself, therefore the movement of the patient's wrist. This data can be used to calculate the motion of the monitored person (overall activity, step count, etc.). Other body worn sensors can sense vital signs like temperature, respiratory rate, pulse and blood pressure without the patient's active contribution or with minimal contribution.

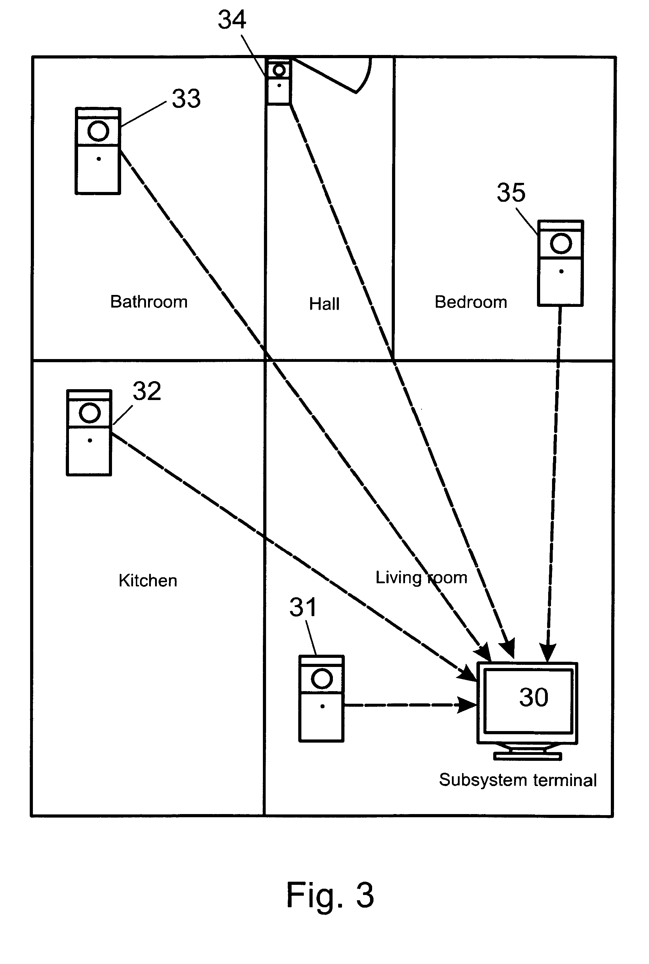

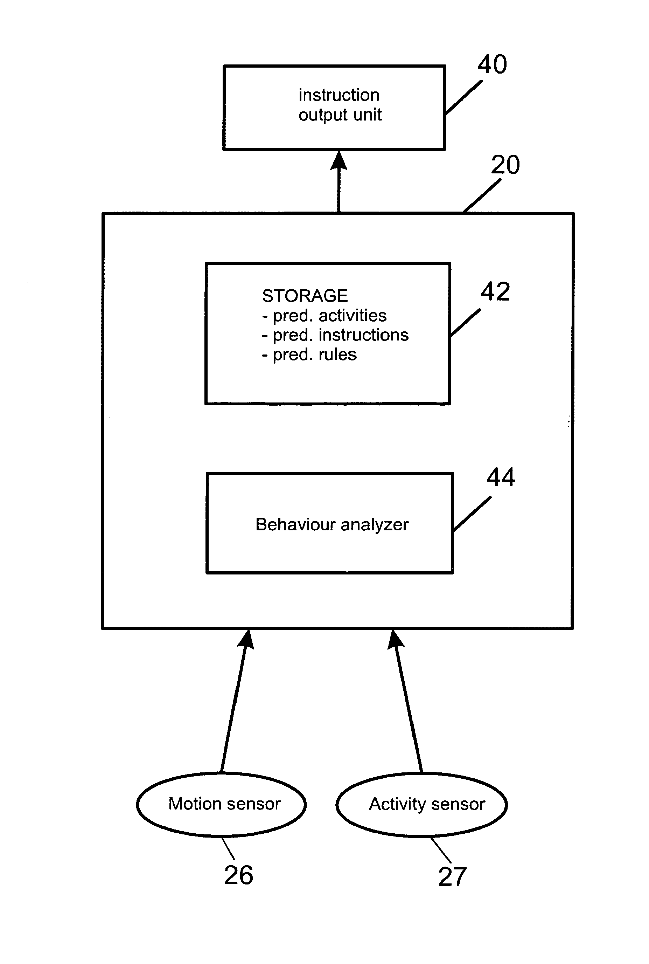

Fig. 3 shows an exemplary arrangement of the elements of a behavioral monitoring subsystem at a location of an individual to be monitored, which is preferably at a distant location from the caregivers and/or the health care specialists and/or the family members. This location may be typically in the home of the individuals to be monitored, who are generally elderly people or patients released from hospital. In this arrangement the subsystem control unit 20 of Fig. 2 is shown as a subsystem terminal 30 with a number of fix mounted sensors 26 (contact sensors and motion sensors) that are connected to the subsystem terminal 30 through a radio or cable communication channel. The contact sensors (not shown) are simple devices, consisting of two matching parts. A contact sensor generally can detect, if these two parts are in contact, or separated, and when a change happens in its state, it sends a signal to the subsystem control unit 20. So e.g. if one part of this sensor is mounted to a door, and the other is to the frame of the door, the sensor can detect if the door is open or closed. Such sensors may be mounted on matching surfaces being relatively moved with respect to each other, such as a door of a room, a door of a piece of furniture or the door of an equipment, such as a refrigerator. In this configuration, each room (e.g. bathroom, hall, bedroom, kitchen and living room) has an own motion detector 31 to 35, which may be preferably wall mounted: This way the system can provide information in which room the monitored person is at a moment. If there are rooms, where there are well separated areas with different, meaningful purpose (living room and kitchen in one common area), more than one motion sensor can be mounted in one room, setup in a way to cover different areas with their field of view. These sensors 31 to 35 provide continuous measurement without any interaction of the individuals to be monitored. They are not in direct contact with the monitored person. The sensors 31 to 35 of the subsystem 21, 22, 23 thus configured provide continuous information regarding to the location of the individual being monitored and further information on closing or opening a door of a room, a piece of furniture or an equipment, that allows conclusions on the current activity of the individuals being monitored. The fix mounted sensors can also be mounted on furniture or household devices (like armchair, bed, cupboard, refrigerator or toilet) in order to monitor patients activity.

The body-worn activity sensors 27 of Fig. 2 that are mounted on a body part of the individuals (not shown) are also connected to the subsystem control unit 20, preferably though a wireless communication channel 27'. These sensors 27 provide continuous information on the activity - such as speed or acceleration of selected body parts - of the individuals to be monitored. The wireless communication link 27' between the body-worn sensors (also called actigraph) 27 and the subsystem control unit 20 may be for example a communication link according to the Bluetooth, Zigbee, Wifi or other standardized specifications. In some cases Zigbee may be preferred because of its flexibility and low power consumption.

The subsystem control unit 20 or home hub may include a general computer provided with a suitable cable and/or radio interface units, a storage unit for storing the measured data, input and output devices for communication with the individuals to be monitored. Such input devices may include for example but not exclusively a keyboard, a pointing device, such as a mouse, a touchpad, or a touch-screen, etc. The output devices may include for example but not exclusively a monitor, preferably a flat screen monitor, a printer, an audio output device, such as a loudspeaker, etc. The interface units typically form integral part of the subsystem control unit, but it is also possible to connect external interface units to the subsystem control unit, e.g. via a USB connector. Such a subsystem control unit is known per se (such as Intel Healthguide) and needs not to be explained further.

As indicated in Fig. 4, an embodiment of the subsystem control unit may also comprise a communication storage 42 for storing
- a set of predetermined activities to be performed by the individuals,
- a set of predetermined instructions relating to the predetermined activities to be communicated to the individual, and
- a set of predetermined rules for each of the activities in order to establish
proper conditions for the activities to be carried out.

A program may also be provided for controlling the communication with the individuals, on the basis of the stored data. The subsystem control unit receives input information from the behavior event monitoring units 26 and 27 especially in the form of a motion sensor, activity sensor etc. Based on motion sensors (and other passive sensors like stove sensor) it is possible to detect what the patient is doing, i.e. the patient started to move in the bedroom in the morning, and moved into a different room, than we can be sure that the patient woke up.
The received behavior event information is processed in a behavioral event analyzing unit 44 for determining the proper timing conditions on the basis of the detected behavioral events. The subsystem control unit 20 may also comprise or be connected to a communication unit or an instruction output unit 40 for transmitting instructions to the individuals according to the determined proper timing conditions.

The proper timing conditions may be determined with such a system as a predetermined time before or after a predetermined event. The patient therefore may be instructed to carry out a scheduled activity a predetermined time before or after a predetermined event. Such an event may be for example: getting up, eating, having a rest, or having a measuring result which needs repetition. The event monitoring elements may be selected from the group of motion sensors, location sensors, activity sensors and the like as shown in Fig. 2 and 3. Scheduling of activities before certain events makes it necessary to determine the expected time of a predetermined event. The expected time of a predetermined event may be predicted on the basis of an event analysis of behavior monitoring data. Event analysis of the behavior monitoring data may include filtering out noise and filtering for certain events, pattern searching and adaption, making conclusions of complex actions based on simple events, etc.

The behavior monitoring may be carried out by detecting behavior specific events of the individual, collecting information relating to the behavior specific events and analyzing the collected information in order to determine a set of regular events or a regular sequence of events that is characteristic for the monitored individual. This may be done in a self learning mode of the system using machine learning algorithms (i.e. neural networks, Bayesian network, fuzzy networks and so on). A detected event may be regarded as a regular event if the event can be detected at substantially the same time or in combination with substantially the same conditions during the daily routine several times wherein the number of repetitions is above a predefined threshold limit. Similarly, a detected sequence of events will be regarded as a regular sequence of events if the sequence can be detected at substantially the same time, substantially with the same order of events or in combination with substantially the same conditions during the daily routine several times wherein the number of repetitions is above a predefined threshold limit. The regular events, the regular sequence of events and other conditions of the events will be stored for further evaluating and use for determining the proper timing conditions.

On the other hand, the irregular event and the irregular sequences of event may not be used for determining the proper timing conditions. A detected event will be regarded as an irregular event and excluded from the regular events if the event can be detected at a substantially different time or in combination with substantially different conditions during the daily routine only once or with a repetition number below the threshold limit. Similarly, a detected sequence of events will be regarded as an irregular sequence of events and excluded from the regular sequence of events if the sequence can be detected at a substantially different time or with substantially different order of events or in combination with substantially different conditions during the daily routine only once or with a repetition number below the threshold limit.

The following two examples will make it even more clear how the method and the system can be used in practice in order to determine the proper timing conditions.

### Example 1

In case of blood glucose measurement there are several approaches. According to a fasting blood sugar measurement (FBS) the patient should not eat for at least 8 hours. This is normally the case after getting up and before the first meal, e.g. breakfast. When carrying out a 2-hour postprandial blood sugar measurement, the patient is not allowed eat for at least 2 hours before the measurement. Another possibility would be to take random blood sugar (RBS) measurements, where several random measurements may be taken throughout the day.

To ensure the proper timing of blood sugar measurement e.g. in case of fasting blood sugar measurement, when the system detects that the patient woke up, the system warns the patient to perform the measurement. In other example when the patient goes to the kitchen and opens the door of the fridge, or starts to cook, the system warns the patient to perform the blood glucose measurement before starting to eat. Similarly, the starting of meals can be detected in case of 2-hour postprandial blood sugar measurement, and the system can warn the patient to make the measurement at the proper time.

### Example 2

In case of blood pressure measurement, the patient has to rest at least for five minutes before taking readings. The system checks the behavioral data, and if the patient's condition is appropriate, can notify the patient to take the measurement.

In the proposed system it is possible to set any arbitrary rule to determine the timing of measurements based on behavior data (or any other data) if the physician has any other unique idea to do so.

Monitored events of a patient may also include activities performed by the patient, such as taking medicine, performing measurements, making exercises without notification by the system. After analyses, the system can determine whether such a self initiated activity is within an acceptable timing period and therefore the activity can be accepted as being carried out under proper timing conditions without repetition. The analyses however can also come to the conclusion that the self initiated activity is outside an acceptable timing period and therefore the activity cannot be accepted as being carried out under proper timing conditions. In this latter case the patient will be reminded by the system to carry out (repeat) the activity under the proper timing conditions determined by the system.

The system can also handle anticipated events, such as expected visit of a nurse or a doctor who is going to assist a specific activity, such as perform a measurement. Practically this is the opposite of the normal operation of the system, where the proper timing conditions have to be determined. In this case the time of the activity is predetermined and the proper conditions have to be guaranteed. In order to achieve the proper conditions for a predetermined event at a predetermined time, the system can notify the patient of the expected assisted activity and give proper instructions in order to ensure the proper condition at the time of the doctor's or nurse's arrival. The system can, in such a case instruct the patient what to do and what not to do in a predetermined interval before arrival of the doctor or the nurse.
In case a measurement needs to be performed less frequently, it might be more cost effective, if the nurse, who visits the patient, brings the measurement device, and helps to perform the measurement. The system can inform the patient about the planned arrival of the nurse, and remind the patient about the desired conditions of the measurement. Here, based on the behavior data, the system warns the patient in case the patient is apparently up to violate the desired condition of the measurement. E.g. a nurse arrival is planned for today to perform yearly fasting blood glucose measurement, but the patient after waking up goes to the fridge and opens its door. At this moment the system warns the patient about the nurse's arrival, and reminds the patient about the desired conditions of measurement.

As suggested in the different embodiments, the patient activities, such as medication performing measurements, doing exercises, etc., are not scheduled according to a time-based plan, they are determined individually and in conformity with the behavioral pattern of the individual. Thus, the proposed system and method is more flexible and satisfies better the individual needs of the patients. Medical professionals using this method and system will be able to use a behavior data based schedule of activities, and therefore make sure that the patient activities are always carried out at the proper time and under the proper conditions.

This written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. Method for communication with an individual to be monitored at a location distant from medical assistance, such as in a home environment, in order to remind the individual of activities relating to medication and/or carrying out measurements or other activity, such as rehabilitation exercises, comprising the steps of:
- providing a set of predetermined activities to be performed by the assisted individuals,
- providing a set of predetermined instructions relating to the predetermined activities to be communicated to the assisted individual,
- providing a set of predetermined rules for each of the activities in order to establish proper conditions for the activities to be carried out,
- continuous monitoring the behavior of the individuals for determining behavioral events in their daily routine,
- determining the proper timing conditions of the predetermined activities with relation to the behavioral events and
- communicating the instructions to the individuals according to the determined proper timing conditions.

2. The method of claim 1, wherein proper timing conditions are determined to be a predetermined time after a predetermined event.

3. The method of claim 2, wherein the individual is instructed to carry out a scheduled activity a predetermined time after a predetermined event.

4. The method of claim 2, wherein the behavioral events are determined by event monitoring elements selected from the group of motion sensors, location sensors, activity sensors and the like.

5. The method of claim 1, wherein proper timing conditions are determined to be a predetermined time before a predetermined event.

6. The method of claim 5, wherein the individual is instructed to carry out a scheduled activity a predetermined time before a predetermined event.

7. The method of claim 5, wherein the expected time of a predetermined event will be predicted on the basis of an event analysis of behavior monitoring data.

8. The method of claim 7, wherein the behavior monitoring is carried out by detecting behavior specific events of the individual, collecting information relating to the behavior specific events and analyzing the collected information in order to determine a set of regular events or regular sequence of events that is characteristic for the monitored individual.

9. The method of claim 8, wherein, a detected event will be regarded as a regular event if the event can be detected at substantially the same time or in combination with substantially the same conditions during the daily routine several times wherein the number of repetitions is above a predefined threshold limit.

10. The method of claim 8, wherein, a detected sequence of events will be regarded as a regular sequence of events if the sequence can be detected at substantially the same time, substantially with the same order of events or in combination with substantially the same conditions during the daily routine several times wherein the number of repetitions is above a predefined threshold limit.

11. The method of claim 8, wherein the regular events, the regular sequence of events and other conditions of the events will be stored for further evaluating.

12. The method of claim 8, wherein, a detected event will be regarded as an irregular event and excluded from the regular events if the event can be detected at a substantially different time or in combination with substantially different conditions during the daily routine only once or with a repetition number below the threshold limit.

13. The method of claim 8, wherein, a detected sequence of events will be regarded as an irregular sequence of events and excluded from the regular sequence of events if the sequence can be detected at a substantially different time or with substantially different order of events or in combination with substantially different conditions during the daily routine only once or with a repetition number below the threshold limit.

14. System for communication with an individual to be monitored at a location distant from medical assistance, such as in a home environment, in order to remind the individual of prescribed activities relating to medication and/or carrying out measurements, comprising:
- a plurality of subsystems (11 ... 13) at the location of the individuals to be monitored, the subsystems comprising a subsystem control unit (20) and at least one behavioral event monitoring unit (26, 27) for collecting information from and relating to the monitored person, with at least a part of the monitoring units being capable of communicating with the subsystem control unit (20),
- a central data server station (10) being capable of communicating with the subsystems (11 - 13), and
- monitoring side terminals (14, 15, 16) being capable of communicating with the central station (10) for providing information for health care professionals and/or care giving personnel and/or authorized family members,
- the subsystem control unit (20) further comprising a communication storage (42) for storing
- a set of predetermined activities to be performed by the individuals,
- a set of predetermined instructions relating to the predetermined activities to be communicated to the individual, and
- a set of predetermined rules for each of the activities in order to establish proper conditions for the activities to be carried out,
- a program for controlling the communication with the individuals, on the basis of the stored data, **characterized in that** the system further comprises:
- at least one behavioral event analyzing unit (44) for determining the proper timing conditions on the basis of the detected behavioral events and
- at least one communication unit (40) for transmitting instructions to the individuals according to the determined proper timing conditions.

15. The system of claim 14, wherein the behavioral event monitoring unit comprises at least one event monitoring element selected from the group of motion sensors, location sensors, activity sensors and the like.
